(19) Europäisches Patentamt
European Patent Office
Office européen des brevets

(11) EP 2 635 705 B1

(12) **FASCICULE DE BREVET EUROPEEN**

(45) Date de publication et mention de la délivrance du brevet:
**05.08.2015 Bulletin 2015/32**

(21) Numéro de dépôt: **11799751.0**

(22) Date de dépôt: **03.11.2011**

(51) Int Cl.:
***C12Q 1/68*** *(2006.01)*

(86) Numéro de dépôt international:
**PCT/FR2011/052571**

(87) Numéro de publication internationale:
**WO 2012/059695 (10.05.2012 Gazette 2012/19)**

(54) **MÉTHODE DE DÉPISTAGE DU CANCER COLORECTAL**

VERFAHREN ZUM KOLOREKTALKREBS-SCREENING

METHOD OF SCREENING FOR COLORECTAL CANCER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.11.2010 FR 1059100**

(43) Date de publication de la demande:
**11.09.2013 Bulletin 2013/37**

(73) Titulaires:
- **Assistance Publique Hôpitaux De Paris 75004 Paris 4 (FR)**
- **Universite Paris 12 - Val De Marne 94000 Creteil (FR)**

(72) Inventeurs:
- **ROPERCH, Jean-Pierre 78270 Lommoye (FR)**
- **SOBHANI, Iradj 94100 Saint Maur des Fosses (FR)**

(74) Mandataire: **Cabinet Plasseraud 52, rue de la Victoire 75440 Paris Cedex 09 (FR)**

(56) Documents cités:
**WO-A2-2007/118704 WO-A2-2009/095596 WO-A2-2010/089538 US-A1- 2008 064 029 US-A1- 2009 053 706**

- **ANG PEI WOON ET AL: "Comprehensive profiling of DNA methylation in colorectal cancer reveals subgroups with distinct clinicopathological and molecular features", BMC CANCER, BIOMED CENTRAL, LONDON, GB, vol. 10, no. 1, 21 mai 2010 (2010-05-21), page 227, XP021075066, ISSN: 1471-2407, DOI: DOI:10.1186/1471-2407-10-227 -& ANG PEI WOON ET AL: "Comprehensive profiling of DNA methylation in colorectal cancer reveals subgroups with distinct clinicopathological and molecular features: Additional file 3", Biomed Central , 21 mai 2010 (2010-05-21), XP002636207, Extrait de l'Internet: URL:http://www.biomedcentral.com/1471-2407/10/227/additional/ [extrait le 2011-05-10]**
- **BIBIKOVA MARINA ET AL: "High-throughput DNA methylation profiling using universal bead arrays", GENOME RESEARCH, COLD SPRING HARBOR LABORATORY PRESS, WOODBURY, NY, US, vol. 16, no. 3, 1 mars 2006 (2006-03-01), pages 383-393, XP002445638, ISSN: 1088-9051, DOI: DOI:10.1101/GR.4410706**
- **BELSHAW N J ET AL: "Profiling CpG island field methylation in both morphologically normal and neoplastic human colonic mucosa", BRITISH JOURNAL OF CANCER, vol. 99, no. 1, juillet 2008 (2008-07), pages 136-142, XP002636202, ISSN: 0007-0920**
- **BIN LEE BO ET AL: "Aberrant Methylation of APC, MGMT, RASSF2A, and Wif-1 Genes in Plasma as a Biomarker for Early Detection of Colorectal Cancer", CLINICAL CANCER RESEARCH, vol. 15, no. 19, octobre 2009 (2009-10), pages 6185-6191, XP002636203, ISSN: 1078-0432**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- KIM YOUNG-HO ET AL: "Epigenomic Analysis of Aberrantly Methylated Genes in Colorectal Cancer Identifies Genes Commonly Affected by Epigenetic Alterations", ANNALS OF SURGICAL ONCOLOGY , 5 février 2011 (2011-02-05), pages 1-10, XP002636204, Extrait de l'Internet: URL: http://www.springerlink.com/content/hx l6004224r076v6/ [extrait le 2011-05-10]

SEQUENCE LISTING

<110>

<120> Methode de dépistage du cancer colorectal

<130> BFF100322

<160> 20

<170> PatentIn version 3.3

<210> 1
<211> 95
<212> DNA
<213> Homo sapiens

<400> 1

cgcggcgagg aagctccata aaagccctgt cgcgacccgc tctctgcacc ccatccgctg 60

gctctcaccc ctcggagacg ctcgcccgac agcat 95

<210> 2
<211> 110
<212> DNA
<213> Homo sapiens

<400> 2

cgggtagacg tttttaaatt cgtttagtaa agataagttt cgggtaaatt tatttacgtt 60

gacgttgttc ggatggagta ggttaattgg aaaagtcggg tttagatacg 110

<210> 3
<211> 69
<212> DNA
<213> Homo sapiens

<400> 3

tctgacggcg ccaggttgcg taggtgcggc acgaggagtt ttcccggcag cgaggaggtc 60

ctgagcagc 69

<210> 4
<211> 76
<212> DNA
<213> Homo sapiens

<400> 4

gggatggaaa gaatcctatg cctggtgaag gtcaagggtt ctcataacct acagagaatt 60

tggggtcagc ctgtcc 76

<210> 5
<211> 95
<212> DNA
<213> Homo sapiens

<400> 5

cgcggcgagg aagttttata aaagttttgt cgcgattcgt tttttgtatt ttattcgttg 60

gtttttattt ttcggagacg ttcgttcgat agtat 95

<210> 6
<211> 110
<212> DNA
<213> Homo sapiens

<400> 6

```
cgggtagacg tttttaaatt cgtttagtaa agataagttt cgggtaaatt tatttacgtt     60
gacgttgttc ggatggagta ggttaattgg aaaagtcggg tttagatacg               110
```

<210> 7
<211> 69
<212> DNA
<213> Homo sapiens

<400> 7

```
tttgacggcg ttaggttgcg taggtgcggt acgaggagtt ttttcggtag cgaggaggtt     60
ttgagtagt                                                             69
```

<210> 8
<211> 76
<212> DNA
<213> Homo sapiens

<400> 8

```
gggatggaaa gaattttatg tttggtgaag gttaagggtt tttataattt atagagaatt     60
tggggttagt ttgttt                                                     76
```

<210> 9
<211> 19
<212> DNA
<213> Artificial

<220>
<223> Amorce Sens

<400> 9
tttgacggcg ttaggttgc 19

<210> 10
<211> 24
<212> DNA
<213> Artificial

<220>
<223> Amorce antisens

<400> 10
actactcaaa acctcctcgc tacc 24

<210> 11
<211> 16
<212> DNA
<213> Artificial

<220>
<223> Sonde TaqMan

<400> 11
cggtacgagg agtttt 16

<210> 12
<211> 20
<212> DNA
<213> Artificial

<220>
<223> Amorce sens

<400> 12
cgcggcgagg aagttttata 20

<210> 13
<211> 23
<212> DNA
<213> Artificial

<220>
<223> Amorce antisens

<400> 13
atactatcga acgaacgtct ccg 23

<210> 14
<211> 18
<212> DNA
<213> Artificial

<220>
<223> Sonde TaqMan

<400> 14
cgcgattcgt tttttgta 18

<210> 15
<211> 21
<212> DNA
<213> Artificial

<220>
<223> Amorce sens

<400> 15
cgggtagacg tttttaaatt c 21

<210> 16
<211> 21
<212> DNA
<213> Artificial

<220>
<223> Amorce antisens

<400> 16
cgtatctaaa cccgactttt c 21

<210> 17
<211> 18
<212> DNA
<213> Artificial

<220>
<223> Sonde TaqMan

<400> 17
cgttgacgtt gttcggat 18

<210> 18
<211> 22
<212> DNA
<213> Artificial

<220>
<223> Sonde amorce

<400> 18
gggatggaaa gaattttatg tt 22

<210> 19
<211> 22
<212> DNA
<213> Artificial

<220>
<223> Amorce antisens

<400> 19
aaacaaacta accccaaatt ct 22

<210> 20
<211> 17
<212> DNA
<213> Artificial

<220>
<223> Sonde TaqMan

<400> 20
agggttttta taattta 17

## Description

La présente invention concerne une méthode de dépistage du cancer colorectal chez un sujet telle que définie dans les revendications.

Le cancer colorectal est une maladie très répandue qui représente 10,5% des cancers dans le monde, et est en forte progression (25000 nouveaux cas par an dans les années 1970, 40000 nouveaux cas par an dans les années 2000 en France). L'espérance de vie des malades reste faible, et est évaluée à moins de 50% à 5 ans tous stades confondus. C'est une maladie fréquente, grave et coûteuse qui relève dans la plupart des pays d'une action de dépistage systématique des personnes à risque moyen (asymptomatique de plus de 50 ans).

Hémoccult est le test actuellement le plus utilisé pour le dépistage du cancer colorectal. Ce test consiste en la recherche de sang occulte dans les selles. Le dépistage comporte alors deux étapes: réalisation du test Hemoccult sur trois échantillons de selles consécutives pour détecter la détection de sang occulte, suivi, en cas de positivité de celui-ci, d'une coloscopie pour détecter des tumeurs colo-rectales. On reconnaît deux limites à ce test : les faux positifs (50%) entraînant des coloscopies inutiles, et les faux négatifs, laissant échapper à la coloscopie des malades porteurs de tumeurs. Le degré de positivité du test dans la population asymptomatique de 50 à 75 ans est de 2 à 3%. Sa valeur prédictive positive, en référence à une coloscopie, est de 50%.

Ainsi, dans les conditions actuelles du dépistage, près de 50% des coloscopies réalisées sont normales. D'autre part, près de 50% des sujets porteurs d'une tumeur colique, bénigne ou maligne, ne sont pas identifiés comme tels par cette stratégie de dépistage.

Ce test (Hemoccult) est critiquable puisqu'il n'est pas spécifique et peu sensible. De plus, en raison des réticences à manipuler les selles, une partie des personnes cibles renonce à subir un tel test de dépistage. Il existe donc aujourd'hui un besoin pour un test spécifique, sensible, non invasif, permettant la détection chez un sujet d'une prédisposition ou d'un risque de cancer colorectal.

Des études récentes ont mis en lumière l'intérêt de la détection de la méthylation comme marqueur diagnostic ou pronostic de différents cancers. On entend par "diagnostic" la détermination d'une affection d'une personne atteinte par une pathologie donnée, et par "pronostic" on entend le degré de gravité et l'évolution ultérieure d'une pathologie. La méthylation est un mécanisme de contrôle naturel qui régule l'expression génique de l'ADN. Par exemple, une méthylation anormale de certains gènes peut diminuer l'expression génique et être associée au développement de cancers. Il est aujourd'hui admis que l'hyperméthylation des îlots CpG présents au niveau des promoteurs de gènes suppresseurs de tumeurs joue un rôle fondamental dans l'initiation et la progression des pathologies cancéreuses.

La demande de brevet FR 2 927 091 décrit un kit de dépistage du cancer colorectal basé sur la détection de l'hyperméthylation d'un marqueur. Cependant, ce test se base sur un unique marqueur et ne présente pas une sensibilité acceptable.

Ang et al, "Comprehensive profiling of DNA methylation in colorectal cancer reveals subgroups with distinct clinicopathological and molecular features.", BMC Cancer, 2010 May 21;10:227, divulgue 202 gènes dont le niveau de méthylation corrèle avec le cancer colorectal, y compris les gènes NPY et PENK.

De façon surprenante, les inventeurs ont réussi à mettre en oeuvre un test moléculaire permettant d'identifier plusieurs marqueurs d'intérêt dont l'hyperméthylation est spécifique des cancers colorectaux. Après de longues recherches, les inventeurs ont mis au point une méthode permettant d'analyser dans les effluents biologiques l'ADN provenant de cellules tumorales intestinales. Il est ainsi possible d'étudier les anomalies fonctionnelles de cet ADN. Ces recherches ont permis de mettre en lumière de nouveaux marqueurs spécifiques des cancers colorectaux.

L'invention se rapporte donc à un test moléculaire se basant sur le concept de Methylation Specific PCR (MSP) en mode multiplex, soit MSPM. La MSPM présente pour intérêt majeur la diminution du nombre de PCR à mettre en oeuvre par rapport au mode monoplex. Ainsi, le mode multiplex permet un gain de temps, car il est plus rapide que plusieurs monoplex, et est économiquement avantageux. L'utilisation de la technique MSPM présente également un intérêt indéniable lorsque l'échantillon biologique choisi pour la mise en oeuvre du test génétique est le sang, puisque la quantité d'ADN circulant dans le sang est relativement faible.

Par **"PCR Multiplex",** on entend une forme de PCR, généralement une PCR quantitative permettant l'amplification simultanée de plusieurs cibles d'intérêt en une seule étape, en utilisant une ou plusieurs amorces spécifiques. Cette technique est très avantageuse pour déterminer la présence de délétions, mutations, polymorphismes ou hyperméthylations de plusieurs marqueurs.

Par opposition, l'expression **"PCR Monoplex"** fait référence à une forme de PCR, généralement une PCR quantitative, permettant l'amplification d'une seule cible d'intérêt.

Par **"méthylation spécifique PCR",** on entend une technique classique de l'art antérieur permettant de mesurer le degré de méthylation d'un gène. Cette technique se base sur le principe de la PCR quantitative. Typiquement, cette technique repose sur le traitement avec le bisulfite de sodium de l'échantillon d'ADN à étudier. Ce traitement permet de transformer chacune des cytosines non méthylées en uraciles dans l'ADN traité. L'échantillon ainsi traité subit ensuite une PCR avec des amorces spécifiques des gènes à traiter. La détermination de la nature des amorces spécifiques

dépend de la séquence nucléotidique à amplifier. Dans le cadre de cette invention, la méthylation spécifique PCR est préférentiellement mise en oeuvre en mode multiplex, on parle alors de Methylation Specific PCR en mode multiplex, soit MSPM.

Ainsi, une méthode de dépistage du cancer colorectal chez un sujet est décrite comprenant une étape de mesure du degré de méthylation d'au moins un des gènes choisi parmi les gènes NPY, PENK et leurs fragments ou variants dans un échantillon biologique obtenu à partir dudit sujet. Par **"dépistage du cancer colorectal",** on entend la détection d'une prédisposition à développer un cancer colorectal, mais aussi la détection d'un cancer colorectal déjà installé chez un sujet.

Par **"méthylation"** on entend l'addition d'un groupement méthyl au niveau du carbone 5 d'une cytosine dans un dinucléotide CpG. Ces dinucléotides sont peu fréquents dans la structure de l'ADN, sauf dans les "îlots" CpGs. Ces îlots sont typiquement représentés au niveau de la région promotrice des gènes. Ainsi, lorsqu'on parle de méthylation d'un gène, on fait référence à la méthylation de la région promotrice dudit gène. La présence d'un groupement méthyle en un site précis empêche l'interaction entre le gène et les facteurs de transcription. Typiquement, les groupes méthyles empêchent les facteurs de transcription de se fixer sur le site d'amplification et au promoteur, et à l'ARN polymérase de se fixer sur le site d'initiation. Aussi, la méthylation de la région promotrice a pour conséquence la répression de la transcription de l'ADN. L'expression **"méthylation d'un gène"** englobe la méthylation des îlots CpG de la séquence nucléotidique du gène mais également la méthylation des séquences nucléotidiques du promoteur du gène auquel cette expression s'applique.

Par **"gène NPY",** on entend le gène qui code pour le neuropeptide Y ou NPY.

Par **"gène PENK",** on entend le gène qui code pour la neuropeptide proenképhaline A ou PENK.

Ces deux neuropeptides sont impliqués dans la prise alimentaire, l'absorption des nutriments et l'équilibre de la dépense énergétique de l'organisme. Ce sont des neuropeptides régulateurs des fonctions digestives qui peuvent également réguler la prise alimentaire et le comportement neuro-digestif.

Par **"fragment d'un gène",** on entend une séquence dudit gène d'une longueur d'au moins 50 paires de bases, préférentiellement d'une longueur comprise entre 60 à 110 paires de bases.

Par "variant d'un gène", on entend une séquence d'acide nucléique présentant un pourcentage d'identité d'au moins 80 %, de préférence d'au moins 90 %, de façon plus préférée d'au moins 98 % par rapport audit gène ou à un fragment dudit gène.

Par "pourcentage d'identité" entre deux séquences d'acides nucléiques ou au sens de la présente invention, on entend désigner un pourcentage de nucléotides entre les deux séquences à comparer, obtenu après le meilleur alignement, ce pourcentage étant purement statistique et les différences entre les deux séquences étant réparties au hasard et sur toute leur longueur. On entend désigner par "meilleur alignement" ou "alignement optimal", l'alignement pour lequel le pourcentage d'identité déterminé comme ci-après est le plus élevé. Les comparaisons de séquences entre deux séquences d'acides nucléiques sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981), au moyen de l'algorithme d'homologie locale de Neddleman et Wunsch (1970), au moyen de la méthode de recherche de similarité de Pearson et Lipman (1988), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI). Afin d'obtenir l'alignement optimal, on utilise de préférence le programme BLAST, avec la matrice BLOSUM 62.

Le pourcentage d'identité entre deux séquences d'acides nucléiques est déterminé en comparant ces deux séquences alignées de manière optimale, la séquence d'acides nucléiques à comparer pouvant comprendre des additions ou des délétions par rapport à la séquence de référence pour un alignement optimal entre ces deux séquences. Le pourcentage d'identité est calculé en déterminant le nombre de positions identiques pour lesquelles le nucléotide est identique entre les deux séquences, en divisant ce nombre de positions identiques par le nombre total de positions comparées et en multipliant le résultat obtenu par 100 pour obtenir le pourcentage d'identité entre ces deux séquences.

Par "variants présentant un pourcentage d'identité d'au moins 80 %, de préférence d'au moins 90 %, de façon plus préférée d'au moins 98 %", on entend désigner les séquences nucléiques présentant, par rapport à la séquence nucléique de référence, certaines modifications comme en particulier une délétion, une troncation, un allongement, une fusion chimérique, et/ou une substitution, notamment ponctuelle, et dont la séquence nucléique présente au moins 80 %, de préférence au moins 90 %, de façon plus préférée au moins 98 %, d'identité après alignement optimal avec la séquence nucléique de référence.

Dans un mode de réalisation particulier, la méthode de dépistage du cancer colorectal décrite comprend une étape de mesure du degré de méthylation d'au moins une des séquences polynucléotidiques choisie parmi SEQ ID N°1 et SEQ ID N°2. La séquence SEQ ID N° 1 correspond à un fragment de la séquence du gène NPY. Quant à la séquence SEQ ID N°2, elle correspond à un fragment de la séquence du gène PENK.

Par **"sujet",** on entend un individu sain ou susceptible d'être atteint d'un cancer ou en quête de dépistage, de diagnostic ou de suivi.

Par **"échantillon biologique",** on entend un échantillon contenant de la matière biologique. Plus préférentiellement, on entend tout échantillon contenant du matériel génétique, tel que des acides nucléiques. Cet échantillon peut provenir d'un prélèvement biologique effectué chez un être vivant (patient humain, animal, végétal). Préférentiellement, les échantillons biologiques selon l'invention sont choisis parmi tous les effluents obtenus à partir d'un sujet humain, par exemple le sang total, le plasma, le sérum, la lymphe, les urines, la salive, les selles, ou la sueur.

Par **"matériel nucléique",** on entend toute séquence d'acide désoxyribonucléique (ADN) ou ribonucléique (ARN). Préférentiellement, l'échantillon biologique est soumis à une étape d'extraction du matériel nucléique avant l'étape de mesure du degré de méthylation. Cette extraction du matériel nucléique s'effectue selon les techniques classiques, bien connues de l'homme du métier. Par exemple, cette extraction peut être effectuée par lyse cellulaire des cellules issues de l'échantillon biologique. Cette lyse peut être de nature chimique, physique, thermique. Cette lyse cellulaire est généralement suivie d'une étape de purification permettant de séparer et concentrer les acides nucléiques d'autres débris cellulaires. Pour la mise en oeuvre de cette extraction et cette purification, les kits commerciaux de type QIAGEN et Zymo Research peuvent être utilisés. Bien entendu, les techniques pertinentes diffèrent en fonction de la nature de l'échantillon biologique testé. Les connaissances de l'homme du métier lui permettent aisément d'adapter ces étapes de lyse et de purification audit échantillon biologique testé.

Préférentiellement, la méthode décrite comprend en outre une étape de mesure du degré de méthylation du gène Wif1, un de ses fragments ou variants dans un échantillon biologique obtenu à partir dudit sujet. Dans un mode de réalisation particulier, la méthode de dépistage du cancer colorectal décrite comprend une étape de mesure du degré de méthylation de la séquence polynucléotidique SEQ ID N°3. La séquence SEQ ID N°3 correspond à un fragment de la séquence du gène Wif1.

Plus préférentiellement, la mesure du degré de méthylation du gène Wif1 ou un de ses fragments ou variants s'effectue simultanément à la mesure du degré de méthylation d'au moins un des gènes choisi parmi NPY, PENK et leurs fragments ou variants dans un échantillon biologique obtenu à partir dudit sujet.

Le gène Wif1 est un inhibiteur de la voie Wnt. L'élément initiateur de la carcinogénèse colorectale est l'inactivation du gène APC dont la protéine joue un rôle relatif au contrôle de la prolifération, de l'apoptose et de la migration des cellules épithéliales intestinales. Cette inactivation provoque l'activation de certaines voies de signalisation, telles que celle de la WNT/Béta-caténine et la formation d'adénomes précoces. Il est connu que Wif1 est l'un des gènes impliqués dans l'inhibition de la voie de signalisation Wnt. Aussi, l'hyperméthylation du gène Wif1 s'accompagne de la levée d'inhibition de cette voie.

Préférentiellement, la méthode décrite comprend en outre une étape de mesure du degré de méthylation d'un gène de ménage. Préférentiellement, cette étape de mesure du degré de méthylation du gène de ménage s'effectue de manière simultanée à la mesure du degré de méthylation d'au moins un des gènes choisi parmi NPY, PENK et leurs fragments ou variants et/ou de manière simultanée à la mesure du degré de méthylation du gène Wif1 ou un de ses fragments ou variants.

Dans un mode de réalisation particulier, la méthode de dépistage du cancer colorectal décrite comprend une étape de mesure du degré de méthylation de la séquence SEQ ID N°4. La séquence SEQ ID N°4 correspond à un fragment de la séquence du gène de l'albumine.

Par **"gène de ménage",** ou **"gène ménager"** ou **"gène constitutif",** ou encore ***"housekeeping*** **gene",** on entend un gène constitutionnel qui code principalement pour des protéines essentielles aux fonctions cellulaires de base. De manière préférée, l'expression des gènes de ménage décrits est ubiquitaire et stable entre les différents tissus. Préférentiellement le gène de ménage est choisi parmi les gènes codant pour l'albumine, la beta actine, l'alpha actine, beta microglobuline, préférentiellement l'albumine. Ces gènes de ménage présentent un intérêt majeur dans le contexte des méthodes décrites. En effet, le degré de méthylation des gènes NPY, PENK et Wif1 ou leurs fragments ou variants est exprimé par rapport à la méthylation d'un gène de ménage dont l'expression est stable.

Typiquement, dans le cadre de la présente invention, la mesure du degré de méthylation des gènes d'intérêt se fait selon les techniques classiques de l'art antérieur. De manière préférée, cette mesure s'effectue selon la technique de "Methylation Specific PCR".

Typiquement, pour l'amplification du gène Wif1, on utilise l'amorce sens SEQ ID N°9, cette séquence comptant 19 bases et présentant 3 sites CpG, et l'amorce antisens SEQ ID N°10, cette amorce comptant 24 bases et présentant 2 sites CpG. La sonde TaqMan-MGB SEQ ID N°11 compte 16 bases et 2 sites CpG.

Typiquement, pour l'amplification du gène NPY, on utilise l'amorce sens SEQ ID N°12, cette séquence comptant 20 bases et présentant 3 sites CpG, et l'amorce antisens SEQ ID N°13, cette amorce comptant 23 bases et présentant 4 sites CpG. La sonde TaqMan-MGB SEQ ID N°14 compte 18 bases et 3 sites CpG.

Typiquement, pour l'amplification du gène PENK, on utilise l'amorce sens SEQ ID N°15, cette séquence comptant 21 bases et présentant 3 sites CpG, et l'amorce antisens SEQ ID N°16, cette amorce comptant 21 bases et présentant 2 sites CpG. La sonde TaqMan-MGB SEQ ID N°17 compte 18 bases et 3 sites CpG.

Typiquement, pour l'amplification du gène de l'albumine, on utilise l'amorce sens SEQ ID N°18, cette séquence comptant 22 bases et présentant aucun site CpG, et l'amorce antisens SEQ ID N°19, cette amorce comptant 22 bases et présentant aucun site CpG. La sonde TaqMan-MGB SEQ ID N°20 compte 17 bases et aucun site CpG.

Par **"degré de méthylation"** ou **"taux de méthylation",** on entend le pourcentage du nombre de sites CpG méthylées du gène cible par rapport à un standard. Par gène cible, on entend un gène pour lequel on veut mesurer le degré de méthylation. Dans le cadre de la présente invention, ces gènes cibles sont Wif1, NPY, PENK, l'albumine et leurs fragments ou variants. Par standard, on entend la séquence sauvage dudit gène cible ou bien la séquence d'un gène de ménage.

Le calcul du degré de méthylation se fait selon les techniques de quantification bien connues de l'homme du métier. Cette quantification peut être absolue ou relative. De manière préférée, son calcul se fait selon la technique dite du ΔΔCT. Cette méthode utilise une formule arithmétique pour exprimer le degré de méthylation d'un gène cible, en normalisant avec un gène de référence. Tout d'abord, les différences ΔCt entre les valeurs du Ct du gène cible et du gène de référence sont déterminées pour l'échantillon à analyser et l'ADN standard. L'ADN standard est typiquement un ADN universellement méthylé. Il permet la normalisation des degrés de méthylation des gènes. Un tel ADN est par exemple commercialisé par la société Zymo Research sous la référence commerciale D5011. Suite à la modification par un kit approprié, tel que le kit commercial EZ DNA Methylation (réf. D5002, Zymo Research), cet ADN standard est utilisé comme référentiel. Les valeurs des Ct obtenues sur les gènes Albumine, Wif1, NPY et PENK correspondent par défaut à 100% de copies méthylées.

$$\Delta Ct_{\text{échantillon}} = Ct\ (\text{cible}_{\text{échantillon}}) - Ct\ (\text{référence}_{\text{échantillon}})$$

$$\Delta Ct_{\text{standard}} = Ct\ (\text{cible}_{\text{standard}}) - Ct\ (\text{référence}_{\text{standard}})$$

Ensuite, le ΔΔCT entre le standard et l'échantillon est calculé :

$$\Delta\Delta Ct = \Delta Ct_{\text{standard}} - \Delta Ct_{\text{échantillon}}$$

La méthode de calcul des ΔΔCT présume que les efficacités d'amplification par PCR des 2 gènes sont égales à 100%. Ainsi, en d'autres termes, cette méthode de calcul présume que la concentration en produits est doublée à chaque cycle de la phase exponentielle de la PCR. On en déduit donc que le degré de méthylation normalisé d'un gène cible est déterminé par la formule $2^{-\Delta\Delta Ct} = 2^{(\text{Ctgene-Ctalbumine})\text{standard} - (\text{Ctgène - Ctalbumine})\text{échantillon}}$

Cette méthode donne un degré de méthylation relatif en fonction du contrôle positif utilisé (ADN standard). Elle tient en outre compte des variations du nombre de copies du gène de référentiel utilisé. Ces variations sont nécessairement dues aux variations de quantité d'ADN ayant servi à la mise en oeuvre de la PCR. Ainsi, les résultats ne sont pas biaisés par la nature du gène de référence.

De manière préférée, le gène de référence est un gène de ménage. De manière encore plus préférée, il s'agit du gène de l'albumine.

Préférentiellement, la méthode décrite comprend la mesure simultanée des degrés de méthylation :

- ➢ des gènes NPY et PENK ou leurs fragments ou variants respectifs, et/ou
- ➢ des gènes NPY et Wif1 ou leurs fragments ou variants respectifs, et/ou
- ➢ des gènes PENK et Wif1 ou leurs fragments ou variants respectifs.

Plus préférentiellement, la méthode décrite comprend la mesure simultanée des degrés de méthylation :

- ➢ des séquences SEQ ID N°1 et SEQ ID N°2; et/ou
- ➢ des séquences SEQ ID N°1 et SEQ ID N°3; et/ou
- ➢ des séquences SEQ ID N°2 et SEQ ID N°3.

Plus préférentiellement, la méthode décrite comprend la mesure simultanée des degrés de méthylation d'une part des gènes Wif1 et d'un gène de ménage ou leurs fragments ou variants respectifs, et d'autre part des gènes NPY et PENK ou leurs fragments ou variants respectifs. Préférentiellement, le gène de ménage est l'albumine. Les inventeurs ont effectivement démontré que la mise en oeuvre d'une MSP multiplex sur les gènes Wif1 et albumine ou leurs fragments ou variants d'un coté, et les gènes NPY et PENK ou leurs fragments ou variants de l'autre, augmente la sensibilité du

test de dépistage du cancer colorectal.

Ainsi, la mesure simultanée des degrés de méthylation sur deux des gènes choisis parmi NPY, PENK, Wif1 et leurs fragments ou variants permet de récupérer des sujets dépistés comme négatifs selon les techniques classiques de l'art antérieur et pourtant atteints d'un cancer colorectal.

Préférentiellement, la méthode décrite comprend en outre une étape de comparaison des degrés de méthylation à une valeur seuil. Lorsque la somme des degrés de méthylation d'une combinaison de deux gènes choisis dans le groupe constitué de NPY et PENK ou leurs fragments ou variants respectifs, NPY et Wif1 ou leurs fragments ou variants respectifs, et PENK et Wif1 ou leurs fragments ou variants respectifs, est supérieure à cette valeur seuil, on estime que le sujet, à partir duquel l'échantillon testé a été prélevé, est positif pour le cancer colorectal.

De manière préférée, la méthode décrite comprend en outre une étape de comparaison des degrés de méthylation d'une part des gènes Wif1 et d'un gène de ménage, ou leurs fragments ou variants et d'autre part des gènes NPY et PENK ou leurs fragments ou variants à une valeur seuil, ladite valeur seuil étant déterminée en fonction des besoins en termes de spécificité et de sensibilité. Pour l'obtention d'un test acceptable, cette valeur seuil est d'environ 2% Ainsi, il convient de comparer d'une part le degré de méthylation des gènes Wif1 et d'un gène de ménage à une valeur seuil, et d'autre part le degré de méthylation des gènes NPY et PENK à ladite valeur seuil.

Par **"sensibilité",** on entend la proportion de tests positifs chez les sujets atteints de cancer colorectal.

Par **"spécificité",** on entend la proportion de tests négatifs chez les sujets non atteints de cancer colorectal.

Par **"cut-off",** ou **"valeur seuil",** on entend la valeur limite du degré de méthylation des gènes cibles au dessus duquel l'échantillon, et donc le sujet dont celui-ci provient, est considéré comme positif pour le diagnostic du cancer colorectal selon la méthode décrite.

Les inventeurs ont mis en oeuvre de fastidieuses études sur un groupe de 246 sujets, dont 23 présentaient un cancer colorectal déclaré, 44 présentaient un cancer d'un autre type et 179 étaient sains.

La mise en oeuvre de test génique multiplex sur des combinaisons de gènes cibles à partir de sérum des sujets a permis aux inventeurs de déterminer une valeur seuil au dessus de laquelle le sujet est correctement dépisté comme étant positif au cancer colorectal, avec une sensibilité et une spécificité donnée. La sensibilité et la spécificité du test de dépistage sont donc dépendantes de la valeur seuil choisie. Aussi, il convient d'adapter cette valeur seuil en fonction de la sensibilité et spécificité adéquates. Les valeurs de la sensibilité et de la spécificité pertinentes sont fonctions de plusieurs critères, généralement dépendants de la population à diagnostiquer. Préférentiellement, cette valeur seuil est comprise entre 1 et 5%, préférentiellement entre 1 et 3,5%, préférentiellement entre 1,5 et 3%, préférentiellement entre 2 et 3%, et encore plus préférentiellement cette valeur seuil est de 2%.

Les inventeurs ont montré qu'une valeur seuil de 2% permet une sensibilité de 65,2% et une spécificité de 95% du test de dépistage. Si l'on diminue cette valeur seuil, la sensibilité augmente mais la spécificité diminue. Par une approche pas à pas, les inventeurs ont réussi à identifier la valeur seuil la plus pertinente pour le développement d'un test sensible au marqueur génique du cancer colorectal et suffisamment spécifique pour écarter les faux positifs et faux négatifs. Ainsi, en fonction de la population visée et des prédispositions de ladite population au cancer colorectal, le test décrit peut être mis en oeuvre avec des valeurs seuils différentes, et ainsi une sensibilité et une spécificité modifiées. En effet, si la population visée est une population à risque et dont les sujets sont susceptibles de développer un cancer colorectal, il est possible de modifier la valeur seuil pour moduler la sensibilité et la spécificité du test.

Préférentiellement la méthode de l'invention utilise un échantillon biologique choisi parmi le sang total, le plasma sanguin, l'urine et les selles dudit sujet. Plus préférentiellement, ledit échantillon biologique est choisi parmi le sang total, le plasma et l'urine dudit sujet. La méthode de l'invention est donc très avantageuse puisqu'elle s'affranchit de la nécessité d'effectuer des tests sur les selles du sujet. Aussi, la méthode de l'invention est non invasive et non rédhibitoire pour le sujet cherchant à effectuer un test de dépistage du cancer colorectal.

Préférentiellement, la méthode décrite comprend en outre une étape d'investigation du colon dudit sujet. Préférentiellement, cette étape d'investigation correspond à une coloscopie optique. Alternativement, cette étape d'investigation correspond à une coloscopie virtuelle, également appelée "coloscanner". La technique de coloscanner repose sur l'étude du colon par scanner et reconstruction en 3 dimensions de l'image interne du tube digestif.. Cette coloscopie permet de vérifier la présence de cancer colorectal chez le sujet considéré comme positif pour le diagnostic du cancer colorectal selon la méthode décrite.

Préférentiellement, la détection du tissu cancéreux est par la suite confirmée par examen histologique.

## EXEMPLE

### Sélection des gènes candidats

### 1. Matériel biologique et extraction de l'ADN génomique

Dans une étude menée chez l'homme en dehors du contexte de dépistage, les inventeurs ont recueilli les

selles, le sang, les urines avant la coloscopie et procédé à l'extraction de l'ADN dans ces effluents. De l'ADN extrait du tissu a également été obtenu sur muqueuse normale et pathologique (polypes adénomateux, cancer) soit par biopsie endoscopique au cours de la coloscopie soit à partir de la pièce chirurgicale. Ces sujets relevaient d'une coloscopie suite à une consultation spécialisée (donc il s'agit d'une population ayant des troubles fonctionnels ou une anémie sans étiologie évidente). Cette étude avait comme objectif de valider un test moléculaire fécal en comparaison au test Hemoccult.

Au total, 648 patients ont été inclus, et les inventeurs ont analysés 577 prélèvements différents. Plusieurs marqueurs moléculaires (mutations ponctuelles, méthylation) ont été étudiés. Les inventeurs ont privilégié l'approche relative à la méthylation des marqueurs et ont ainsi mis en oeuvre une stratégie de sélection de plusieurs gènes qui ont servi à concevoir un test multiplex (NPY, PENK).

Les prélèvements biologiques (sang, urines, selles), avant l'examen coloscopique, et tissulaires (muqueuse normale, polypes adénomateux, cancer invasif) ont été soumis à une extraction d'ADN par le kit QiAmp midi kit (Qiagen). Il s'agissait de l'ADN de selles provenant de sujets normaux (N=10), sujets avec cancer (N=10).

## 2. Puce de méthylation

La puce de méthylation « Goldengate », commercialisée par la société Illumina (USA), permet d'analyser la méthylation de 1505 sites CpG qui correspondent à 807 gènes dont 28,6% contiennent un site CpG par gène, 57,3% contiennent deux sites CpG par gène et 14,1% contiennent au moins trois sites. Les échantillons d'ADN ont été traités par une solution de bisulfite de sodium, par le kit EZ DNA Methylation (réf. D5002, Zymo Research), puis marqués par deux sondes de fluorescence ciblant distinctement les formes méthylées (béta) et non méthylées (alpha) des brins d'ADN et soumis à l'amplification génique (PCR). Le degré de méthylation du gène étudié a été établi sur la valeur du rapport (béta) / (alpha+béta) allant de 0 (non méthylé) à 1 (totalement méthylé). A chaque valeur communiquée correspondait une valeur p désignant la probabilité d'une mauvaise estimation du degré de méthylation. L'analyse bioinformatique descriptive a été effectuée sur la valeur seuil $p<0.01$.

## 3. Démarche de la sélection des gènes pertinents

La démarche mise en oeuvre par les inventeurs a permis l'identification des cibles pertinentes dans différents effluents biologiques (sang, selles, urines) en référence au tissu (normal et tumoral).

Les comparaisons intergroupes ont été réalisées avec une stratégie basée sur l'identification des meilleurs marqueurs différentiels intergroupes (normaux versus cancers) pour le tissu ou le même effluent biologique d'une part et sur la concordance de signaux entre différents effluents biologiques d'autre part. Sur 1015 gènes retenus comme étant soumis à une possible méthylation, une liste de gènes hyperméthylés (Cancer-Normal>ou=20% avec une valeur $p<0,0001$ au plan technique) a été établie pour le tissu ; elle contenait 146 cibles hyperméthylées.

De la même manière, la liste des gènes hyperméthylés selon le même critère contenait 134 cibles dans les selles et 122 cibles dans le sang. Ensuite l'interclassement des listes entre elles en se basant sur les valeurs individuelles a permis de retenir 34 cibles communes entre le tissu et le sang et 46 cibles entre les selles et le sang. *In fine,* les inventeurs ont identifiés comme pertinents les gènes NPY, PENK et Wif1, et plus particulièrement, les fragments de ces gènes, respectivement SEQ ID N°1, 2 et 3.

Les inventeurs ont ainsi identifiés 3 gènes candidats (Wif1, NPY, et PENK). Les inventeurs ont mis en évidence l'intérêt de ces gènes pour l'élaboration de tests géniques basés sur la réalisation de la Méthylation Spécifique PCR (MSP) multiplex.

## 4. Amplification génique en temps réel par la technique MSP

Les amorces et les sondes TaqMan-MGB (Applied Biosystems) ont été générées en tenant compte de la modification de l'ADN. Le tableau ci-dessous regroupe les séquences des amorces et sondes correspondant au gène de « ménage » : Albumine ainsi que celles de fragments des 3 gènes NPY, PENK et Wif1 (respectivement SEQ ID N°1, 2 et 3).

Le tableau qui suit indique les séquences nucléotidiques des produits d'amplifications du gène de ménage et des gènes cibles.

| | Séquences nucléiques pour PCR-Méthylation Spécifique |
|---|---|
| **NPY** | ADN non modifié (SEQ ID N°1):<br>5'<br>CGCGGCGAGGAAGCTCCATAAAAGCCCTGTCGCGACCCGCTCTCTGCACCCCATCCG CTGGCTCTCACCCCTCGGAGACGCTCGCCCGACAGCAT 3'<br>ADN modifié : Amplicon de 95 bases (SEQ ID N°5)<br>5'<br>CGCGGCGAGGAAGTTTTATAAAAGTTTTGTCGCGATTCGTTTTTTGTATTTTATTCG TTGGTTTTTATTTTTCGGAGACGTTCGTTCGATAGTAT 3' |
| **PENK** | ADN non modifié(SEQ ID N°2):<br>5'<br>CGGGTAGACGTTTTTAAATTCGTTTAGTAAAGATAAGTTTCGGGTAAATTTATTTAC GTTGACGTTGTTCGGATGGAGTAGGTTAATTGGAAAAGTCGGGTTTAGATACG 3'<br>ADN modifié : Amplicon de 110 bases (SEQ ID N°6)<br>5'<br>CGGGTAGACGTTTTTAAATTCGTTTAGTAAAGATAAGTTTCGGGTAAATTTATTTAC GTTGACGTTGTTCGGATGGAGTAGGTTAATTGGAAAAGTCGGGTTTAGATACG 3' |
| **Wif1** | ADN non modifié (SEQ ID N°3) :<br>5'<br>TCTGACGGCGCCAGGTTGCGTAGGTGCGGCACGAGGAGTTTTCCCGGCAGCGAGGA GGTCCTGAGCAGC 3'<br>ADN modifié : Amplicon de 69 bases (SEQ ID N°7)<br>5'<br>TTTGACGGCGTTAGGTTGCGTAGGTGCGGTACGAGGAGTTTTTTCGGTAGCGAGGA GGTTTTGAGTAGT 3 |
| **Albumine** | ADN non modifié (SEQ ID N°4):<br>5'GGGATGGAAAGAATCCTATGCCTGGTGAAGGTCAAGGGTTCTCATAACCTACAGA GAATTTGGGGTCAGCCTGTCC 3'<br>ADN modifié : Amplicon de 77 bases (SEQ ID N°8)<br>5'GGGATGGAAAGAATTTTATGTTTGGTGAAGGTTAAGGGTTTTTATAATTTATAGAG AATTTGGGGTTAGTTTGTTT 3' |

Le tableau ci-dessous indique les séquenles des amorces sens, des amorces antisens et des sondes TaqMan utilisées.

| | Amorce Sens | Amorce Antisens | Sonde TaqMan-MGB |
|---|---|---|---|
| **Wif1** | SEQ ID N°9<br>TTTGACGGCGTTAGGTTGC<br>T°m=58.4°C 19 bases 3 CpG | SEQ ID N°10<br>ACTACTCAAAACCTCCTCGCTACC<br>T°m=58.1°C 24 bases 2 CpG | SEQ ID N°11<br>CGGTACGAGGAGTTTT<br>T°m=70°C 16 bases 2 CpG |
| **NPY** | SEQ ID N° 12<br>CGCGGCGAGGAAGTTTTATA<br>T°m=59°C 20 bases 3 CpG | SEQ ID N°13<br>ATACTATCGAACGAACGTCTCCG<br>T°m=58.4°C 23 bases 4 CpG | SEQ ID N°14<br>CGCGATTCGTTTTTTGTA<br>T°m=68°C 18 bases 3 CpG |
| **PENK** | SEQ ID N° 15<br>CGGGTAGACGTTTTTAAATTC<br>T°m=52.3°C 21 bases 3 CpG | SEQ ID N°16<br>CGTATCTAAACCCGACTTTTC<br>T°m=52.2°C 21 bases 2 CpG | SEQ ID N° 17<br>CGTTGACGTTGTTCGGAT<br>T°m=70°C 18 bases 3 CpG |
| **Albumine** | SEQ ID N° 18<br>GGGATGGAAAGAATTTTATGTT<br>T°m=52.9°C 22 bases 0 CpG | SEQ ID N° 19<br>AAACAAACTAACCCCAAATTCT<br>T°m=52.7°C 22 bases 0 CpG | SEQ ID N°20<br>AGGGTTTTTATAATTTA<br>T°m=63°C 17 bases 0 CpG |

L'amplification est réalisée en plaques 96 puits scellées par un film de qualité optique, compatible qPCR (ABgene). 5,8 $\mu$l d'ADN modifié (10 ng ADN standard et ADN de patients) sont additionnés à la concentration finale de 400 nmol de chaque amorce (sens et antisens) et à la concentration finale de 250 nmol de sonde TaqMan-MGB et de 1X de solution PCR (Applied Biosystems). Le volume réactionnel est de 20 $\mu$l. Les conditions d'amplification sont les suivantes:

- ➢ Hot start : 94°C pendant 15 minutes;
- ➢ Up to 48 cycles : 94°C pendant 15 secondes, étape de dénaturation;
- ➢ 60°C pendant 60 secondes, étapes d'hybridation (annealing) et d'élongation.

L'ADN standard est un ADN qui est universellement méthylé (réf. D5011, Zymo Research). Suite à la modification par le kit EZ DNA Methylation (réf. D5002, Zymo Research), il est utilisé par MSP comme référentiel. Les valeurs des Ct obtenues sur les fragments des gènes Albumine, Wif1, NPY et PENK correspondent par défaut à 100% de copies méthylées.

*a) Pourcentage de méthylation selon la méthode de delta-delta Ct*

Le degré de méthylation est quantifié selon la méthode de $\Delta\Delta Ct = 2^{-(Ctgène-Ctalbumine)échantillon + (Ctgène-Ctalbumine)standard}$. Cette méthode donne une valeur relative du degré de méthylation en fonction du contrôle positif utilisé (ADN standard). Elle tient compte des variations du nombre de copies du gène Albumine qui ne peuvent être dues qu'à une variation de quantités d'ADN ayant servi pour la réalisation de la MSP.

*b) Phase évacuation du Test moléculaire MSPM sur ADN tissulaires*

La réalisation de la phase d'évaluation a fait appel à 32 ADN tissulaires humains incluant 16 ADN tumoraux et 16 ADN tissus autologues dits « normaux ».

| | **cut off = 20 %** | |
|---|---|---|
| **MSP monoplex** | **Sensibilité** | **Spécificité** |
| Wif1 | 68.75% | 100% |
| NPY | 100% | 93.75% |
| PENK | 75% | 93.75% |
| **MSP multiplex** | | |
| NPY + PENK (duplex) | 100% | 93.75% |
| Wif1 + PENK (duplex) | 75% | 100% |
| Wif1+ NPY (duplex) | 100% | 100% |
| **Combinaison de MSP multiplex = 2 duplex par run** | | |
| Wif1 + albumine | 100% | 100% |
| NPY+PENK | | |

*c) Détermination par « Méthylation Spécifique PCR » des performances du Test génique sur ADN sérique*

Les inventeurs ont développé une MSP en mode multiplex à partir de l'ADN tissulaire. Les très bonnes performances du test multiplex affichées en termes de spécificité et de sensibilité sur prélèvements tissulaires ont conduits à procéder à la validation sur un échantillonnage sérique.

*i. Matériel biologique*

Nous avons évalué la pertinence du Test génique multiplex sur un échantillonnage de 246 sérums issus de patients hospitalisés soumis au protocole de test multiplex.

*ii. Extraction de l'ADN sérique: choix du kit d'extraction*

Les inventeurs ont évalué la pertinence du Kit d'extraction d'ADN commercialisé par la société Qiagen, Kit QiAmp midi kit, et le Kit ZR Serum (réf. D3013) commercialisé par la société Zymo Research. Le Kit ZR Serum est celui qui a été retenu. Le volume de 4 ml de sérum (8-10 ml de sang total) est recommandé pour obtenir une quantité suffisante de molécules nucléiques nécessaire à la mise en oeuvre de 4 MSP, correspondant à 2 duplex réalisés en combinaison [(Wif1+Albumine) et (NPY+PENK)].

*d) Validation*

La phase de validation du Test multiplex a été réalisée sur un premier échantillonnage de 246 sérums. Parmi ces 246 sérums, 23 sont issus d'individus présentant un cancer colique ou rectal, 44 sont issus d'individus présentant un autre type de cancer, et 179 sont issus d'individus non atteints de cancer. Les performances, affichées en termes de Spécificité et de Sensibilité, de la MSP multiplex ciblant les 3 gènes candidats mis en association (2 MSP duplex) sont indiquées dans le tableau ci-dessous. Le duplex 1 correspond à une MSP Multiplex de Wif1 et de l'albumine tandis que le duplex 2 correspond une MSP Multiplex de NPY et de PENK.

| **Test génique multiplex sérique** | **cut off = 3%** | | **cut off = 2%** | |
|---|---|---|---|---|
| | **Sensibilité** | **Spécificité** | **Sensibilité** | **Spécificité** |
| **246 sérums dont:**<br>-23 cancers colorectaux<br>-44 autres cancers<br>-179 non cancers | **56,5%** | **98,3%** | **65,2%** | **95%** |

Il apparaît que la spécificité et la sensibilité sont dépendantes de la valeur seuil choisie initialement. Ainsi une valeur seuil (ou cut off) de 3% permet une sensibilité de 56,5% et une spécificité de 98,3%, tandis qu'une valeur seuil de 2% permet une sensibilité de 65,2% et une spécificité de 95%. Ainsi, il convient d'adapter la valeur seuilen fonction de la population d'individus à diagnostiquer.

*e) Comparatif des performances : Test multiplex versus Test monoplex*

La phase de validation du Test multiplex selon l'invention a permis de déterminer les performances de ce test en termes de Sensibilité (65,2 %) (15 cancers colorectaux CCR dépistés sur un total de 23) et de 95 % en termes de Spécificité. Le tableau ci-dessous présente les résultats des 15 individus souffrant d'un cancer colorectal et positifs en mode multiplex et en mode de combinaison de multiplex, comparés aux résultats obtenus en mode monoplex.

| | MSP Monoplex | | | MSP Multiplex (Duplex) | | | Combinaison de MSP Multiplex (duplex 1 + duplex 2) |
|---|---|---|---|---|---|---|---|
| ID | NPY | PENK | Wif1 | Cut off =2% | | | |
| | | | | NPY+ PENK | NPY+ Wif1 | PENK+ Wif1 | |
| CCR1 | 65.33 (+) | 11.35 (+) | 12.79 (+) | + | + | + | + |
| CCR2 | 3.16 (+) | 2.84 (+) | 5.57 (+) | + | + | + | + |
| CCR3 | 38.03 (+) | 4.40 (+) | 100.63 (+) | + | + | + | + |
| CCR4 | 46.37 (+) | 9.89 (+) | 15.19 (+) | + | + | + | + |
| CCR5 | 0 (-) | 0.12 (-) | 2.72 (+) | - | + | + | + |
| CCR6 | 56.07 (+) | 105.85 (+) | 0.07 (-) | + | + | + | + |

(suite)

| ID | MSP Monoplex NPY | MSP Monoplex PENK | MSP Monoplex Wif1 | MSP Multiplex (Duplex) Cut off =2% NPY+ PENK | NPY+ Wif1 | PENK+ Wif1 | Combinaison de MSP Multiplex (duplex 1 + duplex 2) |
|---|---|---|---|---|---|---|---|
| CCR7 | 1.59 (-) | 1.14 (-) | 0.87 (-) | + | + | + | + |
| CCR8 | 40.55 (+) | 2.68 (+) | 19.57 (+) | + | + | + | + |
| CCR9 | 20.92 (+) | 2.3 (+) | 6.37 (+) | + | + | + | + |
| CCR10 | 54.33 (+) | 8.87 (+) | 39.24 (+) | + | + | + | + |
| CCR11 | 0 (-) | 0 (-) | 4.40 (+) | - | + | + | + |
| CCR12 | 7.22 (+) | 0 (-) | 1.92 (-) | + | + | - | + |
| CCR13 | 2.31 (+) | 0.59 (-) | 6.13 (+) | + | + | + | + |
| CCR14 | 4.44 (+) | 0 (-) | 0 (-) | + | + | - | + |
| CCR15 | 1.43 (-) | 0.81 (-) | 0.25 (-) | + | - | - | + |

Le duplex 1 correspond à une MSP Multiplex des fragments de Wif1 et de l'albumine, tandis que le duplex 2 correspond une MSP Multiplex des fragments de NPY et de PENK.
Les inventeurs démontrent ainsi que la réalisation de la MSP multiplex (duplex) augmente la valeur de la sensibilité du test (récupération de sujets atteints de cancer colorectal classés comme négatifs en MSP monoplex).
Les inventeurs ont également démontré que la combinaison de MSP multiplex (notamment la combinaison du duplex 1 avec le duplex 2) offre des résultats encore améliorés. Les 15 individus atteints de cancer colorectal sont tous positifs au test.

## Revendications

**1.** Méthode de dépistage du cancer colorectal chez un sujet , **caractérisée en ce qu'**elle comprend :

- ➢ la mesure simultanée des degrés de méthylation des gènes NPY et PENK ou leurs fragments respectifs représentés en SEQ ID N°1 et SEQ ID N°2, et
- ➢ la mesure simultanée des degrés de méthylation des gènes NPY et Wif1 ou leurs fragments respectifs représentés en SEQ ID N°1 et SEQ ID N°3, et
- ➢ la mesure simultanée des degrés de méthylation des gènes PENK et Wif1 ou leurs fragments respectifs représentés en SEQ ID N°2 et SEQ ID N°3

dans un échantillon biologique obtenu à partir dudit sujet.

**2.** Méthode de dépistage du cancer colorectal chez un sujet, **caractérisée en ce qu'**elle comprend d'une part la mesure simultanée des degrés de méthylation des gènes Wif1 et de l'albumine ou leurs fragments respectifs SEQ ID N°3 et SEQ ID N°4, et d'autre part la mesure simultanée des degrés de méthylation des gènes NPY et PENK ou leurs fragments respectifs SEQ ID N°1 et SEQ ID N°2, dans un échantillon biologique obtenu à partir dudit sujet.

**3.** Méthode selon la revendication 1 ou 2, **caractérisée en ce qu'**elle comprend en outre une étape de comparaison desdits degrés de méthylation à une valeur seuil, ladite valeur seuil étant comprise entre 1,5 et 3%.

**4.** Méthode selon la revendication 3, caractérisée ne ce que ladite valeur seuil est de 2%.

**5.** Méthode selon l'une quelconque des revendications 1 à 4, **caractérisée en ce que** ledit échantillon biologique est choisi parmi le sang total, le plasma, le sérum, la lymphe, les urines, la salive, les selles, ou la sueur..

**6.** Méthode selon l'une quelconque des revendications 1 à 5, **caractérisée en ce que** ledit échantillon biologique est choisi parmi le sang total, le plasma et l'urine dudit sujet.

## Patentansprüche

**1.** Verfahren zum Kolorektalkrebs-Screening bei einer Versuchsperson, **dadurch gekennzeichnet, dass** es umfasst:

- die gleichzeitige Messung der Methylierungsgrade der Gene NPY und PENK oder ihrer jeweiligen Fragmente, dargestellt durch SEQ ID NO:1und SEQ ID NO:2, und
- die gleichzeitige Messung der Methylierungsgrade der Gene NPY und Wif1 oder ihrer jeweiligen Fragmente, dargestellt durch SEQ ID NO:1 und SEQ ID NO:3, und
- die gleichzeitige Messung der Methylierungsgrade der Gene PENK und Wif1 oder ihrer jeweiligen Fragmente, dargestellt durch SEQ ID NO:2 und SEQ ID NO:3,

in einer biologischen Probe, die von der Versuchsperson erhalten wurde.

**2.** Verfahren zum Kolorektalkrebs-Screening bei einer Versuchsperson, **dadurch gekennzeichnet, dass** es einerseits die gleichzeitige Messung der Methylierungsgrade der Wif1-Gene und von Albumin oder ihrer jeweiligen Fragmente SEQ ID NO:3 und SEQ ID NO:4 und andererseits die gleichzeitige Messung der Methylierungsgrade der Gene NPY und PENK oder ihrer jeweiligen Fragmente SEQ ID NO:1 und SEQ ID NO:2 umfasst, in einer biologischen Probe, die von der Versuchsperson erhalten wurde.

**3.** Verfahren nach Anspruch 1 oder 2, **dadurch gekennzeichnet, dass** es weiterhin einen Schritt des Vergleichs der Methylierungsgrade mit einem Schwellenwert umfasst, wobei der Schwellenwert zwischen 1,5 und 3 % umfasst.

**4.** Verfahren nach Anspruch 3, **dadurch gekennzeichnet, dass** der Schwellenwert 2 % ist.

**5.** Verfahren nach einem der Ansprüche 1 bis 4, **dadurch gekennzeichnet, dass** die biologische Probe ausgewählt wird aus Gesamtblut, Plasma, Serum, Lymphe, Urin, Speichel, Stuhl oder Schweiß.

**6.** Verfahren nach einem der Ansprüche 1 bis 5, **dadurch gekennzeichnet, dass** die biologische Probe ausgewählt wird aus Gesamtblut, Plasma und Urin der Versuchsperson.

## Claims

**1.** A method of diagnosing colorectal cancer in a subject, comprising :

• simultaneous measurement of the degrees of methylation of NPY and PENK genes or their fragments thereof as set forth in SEQ ID NO. 1 and SEQ ID No. 2 and
• simultaneous measurement of the degrees of methylation of NPY and Wif1 genes or their fragments thereof as set forth in SEQ ID NO. 1 and SEQ ID No. 3, and

• simultaneous measurement of the degrees of methylation of PENK and Wif1 genes or their fragments thereof set forth in SEQ ID NO. 2 and SEQ ID No. 3

in a biological sample obtained from said subject.

**2.** A method of diagnosing colorectal cancer in a subject, comprising on the one hand the simultaneous measurement of the degrees of methylation of Wif1 and albumin genes or their fragment thereof set forth in SEQ ID NO. 3 and SEQ ID NO. 4, and on the other hand the simultaneous measurement of the degrees of methylation of NPY and PENK genes or their fragments thereof set forth in SEQ ID NO. 1 and SEQ ID No. 2 in a biological sample obtained from said subject.

**3.** The method according to claim 1 or 2, **characterized in that** it further comprises a step of comparing said degrees of methylation with a threshold value, said threshold value being comprised between 1.5 and 3%.

**4.** The method according to claim 3, **characterized in that** said threshold value is 2%.

**5.** The method according to any one of claims 1 to 4, **characterized in that** said biological sample is selected from whole blood, blood plasma, serum, lymph, urine, saliva, feces or sweat.

**6.** The method according to any one of claims 1 to 5, **characterized in that** said biological sample is selected from whole blood, plasma and urine from said subject.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2927091

### Littérature non-brevet citée dans la description

- **ANG et al.** Comprehensive profiling of DNA methylation in colorectal cancer reveals subgroups with distinct clinicopathological and molecular features. *BMC Cancer,* 21 Mai 2010, vol. 10, 227